# EUROPÄISCHE PATENTSCHRIFT

(11) **EP 1 090 612 B1**
(45) Veröffentlichungstag und Bekanntmachung des Hinweises auf die Patenterteilung: **21.07.2004**
(21) Anmeldenummer: 99119818.5
(22) Anmeldetag: 07.10.1999
(51) Int. Cl.: A61F 5/01, A61F 5/14

(54) **Vorrichtung zum Korrigieren einer Fehlstellung einer Zehe**
Device to correct the malposition of the large toe
Dispositif pour corriger la malposition du gros orteil

(43) Veröffentlichungstag der Anmeldung: 11.04.2001
(73) Patentinhaber: Weitzel, Wolfgang, Dr., 89520 Heidenheim (DE)
(72) Erfinder: Weitzel, Wolfgang, Dr., 89520 Heidenheim (DE)
(74) Vertreter: Dr. Weitzel & Partner

(56) Entgegenhaltungen:
- DE-A- 19 541 173
- FR-A- 2 626 170
- US-A- 4 729 369
- US-A- 4 745 927
- US-A- 5 572 808

## Beschreibung

Die Erfindung betrifft eine Vorrichtung zum Korrigieren einer Fehlstellung einer Zehe. Dabei kommt in erster Linie die große Fußzehe in Betracht. Bei dieser kann es zu einer Schiefstellung kommen, die die Bildung eines Ballens verursacht. Die große Zehe ist in diesem Falle nach außen geneigt, derart, daß sie an der nächsten Zehe anliegt. Dies kann zu erheblichen Beschwerden des Patienten führen, die eine Operation notwendig machen. Der nächstliegende Stand der Technik wird durch US-A-5 572 808 dargestellt.

Es gibt sogenannte Nachtschienen. Eine solche Vorrichtung umfaßt Gurte, die an der großen Zehe angreifen und diese mehr oder minder elastisch in die Soll-Stellung verschwenken. Diese Nachtschiene wird nur während der Nacht angelegt. Sie nimmt einen erheblichen Raum ein, so daß sie nicht in einen Schuh paßt. Außerdem ist das eigentliche Korrekturelement starr und erlaubt schon aus diesem Grunde kein Tragen bei Tage.

Der Erfindung liegt die Aufgabe zugrunde, eine Vorrichtung zu schaffen, die auch zum Tragen bei Tage in einer Fußbekleidung geeignet ist, die ein geringes Gewicht hat, kostengünstig herstellbar ist, eine zuverlässige Korrektur erzielt, dem Träger beim Tragen keine oder nur geringe Unannehmlichkeiten verursacht und dauerhaft ist.

Diese Aufgabe wird durch die Merkmale von Anspruch 1 gelöst.

Die Erfindung ist anhand der Zeichnungen näher erläutert.
- Fig. 1: zeigt in Draufsicht sowie in einer Seitenansicht eine erste Ausführungsform einer erfindungsgemäßen Korrekturvorrichtung.

Die Figuren 2 und 3 zeigen analoge Darstellungen einer zweiten bzw. einer dritten Ausführungsform.

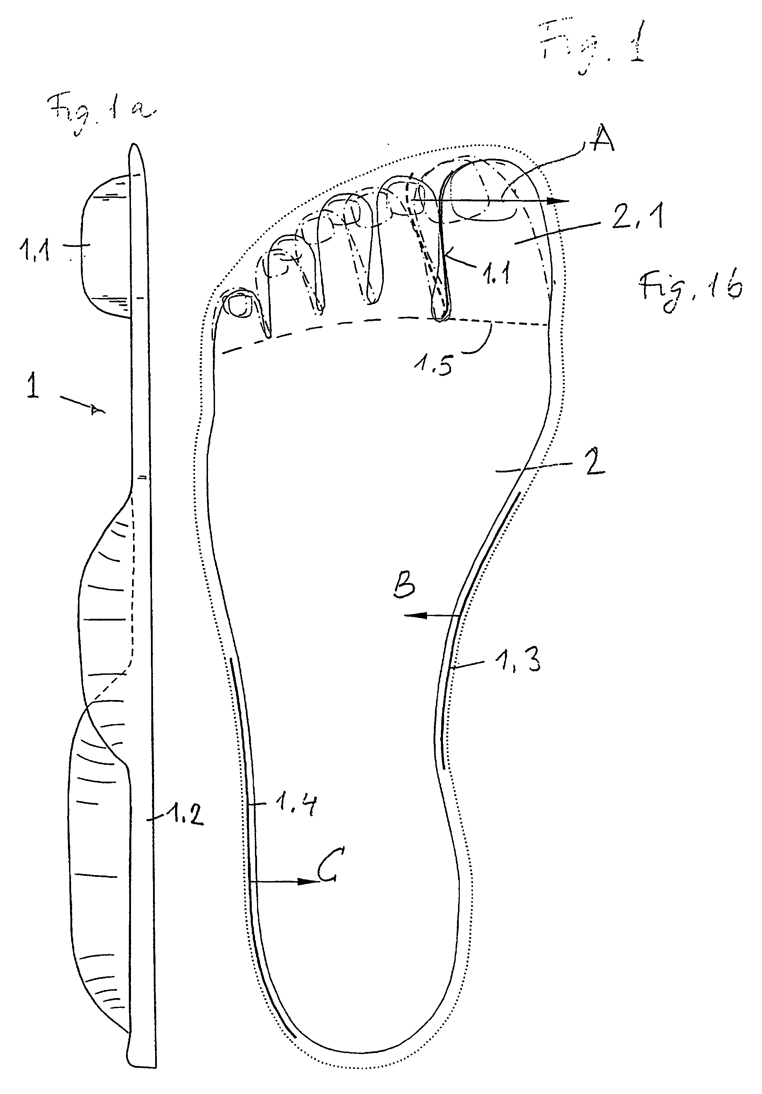

Die in Fig. 1 dargestellte Vorrichtung 1 mit den Figuren 1a und 1b dient zum Korrigieren der Fehlstellung der großen Zehe. Dabei ist in Fig. 1b ein Fuß 2 in seinen Konturen abgebildet, so wie er bei Gebrauch der Vorrichtung 1 zugeordnet ist. Die Zehen sind dabei strichpunktiert in der Fehlstellung dargestellt, und ausgezogen in der korrigierten Stellung. Dabei geht es in erster Linie um die große Zehe 2.1, die in gestrichelter Stellung nach links geneigt ist und die durch den Pfeil A nach rechts in ihre Soll-Stellung verschwenkt werden soll.

Die Korrekturvorrichtung 1 hat eine - punktiert dargestellte - Kontur, die im wesentlichen der Fußkontur entspricht. Sie besteht aus Kunststoff. Sie weist als wichtigstes Element einen Korrekturflansch 1.1 auf. Der Korrekturflansch ist einer Basis 1.2 der Vorrichtung angeformt. Wie man aus Fig. 1b erkennt, ist er in Draufsicht gesehen verhältnismäßig dünn. Er ist derart angeordnet, daß bei Aufsetzen des Fußes 2 auf die Vorrichtung 1 eine mehr oder minder stramme Anlage der Fußzehe 2.1 an den Korrekturflansch 1.1 erfolgt, und die Verschwenkung der Fußzehe 2.1 in Richtung des Pfeiles A erzielt wird. Dabei werden auch die anderen Zehen entlastet, so daß die ausgezogen gezeigten Stellungen einnehmen.

Man erkennt ferner Halte- oder Einspannmittel. Dies sind ebenfalls Flansche 1.3 und 1.4, die der Basis 1.2 angeformt und somit mit dieser einteilig sind. Diese Halteflansche 1.3 und 1.4 geben dem Fuß 2 einen gewissen Halt, indem sie - entgegen der Kraft A - Kräfte B und C auf den Fuß 2 ausüben.

Befindet sich die Vorrichtung 1 in einem Schuh als Einlage, so wird der Fuß durch den Schuh gehalten. Insofern kann auf die Halteflansche 1.3 und 1.4 ganz oder teilweise verzichtet werden.

Es versteht sich, daß die genannten Flansche, Korrekturflansch 1.1 und die beiden Halteflansche 1.3 und 1.4, nicht zwingend mit der Basis 1.2 einteilig sein müssen. Sie können auch eigene Teile darstellen, die auf irgendeine Weise mit der Basis 1.2 fest verbunden sind.

Die gestrichelte Linie 1.5 in Fig. 1b soll folgendes andeuten: Beim Tragen einer Einlage, so wie hier dargestellt, muß sichergestellt sein, daß jedenfalls die Zehen, insbesondere die große Zehe 2.1, eine Abrollbewegung ausführen können. Deswegen muß die Basis an dieser Stelle entsprechend nachgiebig sein. Hier kann ein sogenanntes Filmscharnier vorgesehen werden.

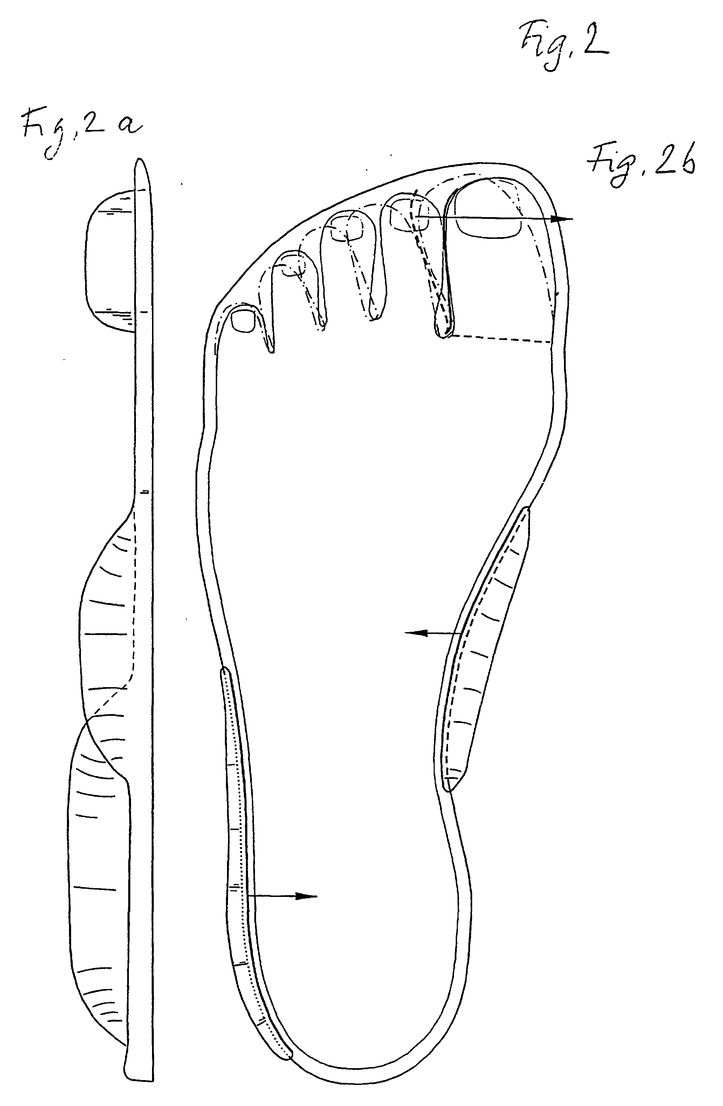

Bei der Ausführungsform gemäß Fig. 2 sind die Halteflansche 1.3, 1.4 etwas verstärkt ausgebildet. Sie können als Polster für den Fuß 2 dienen.

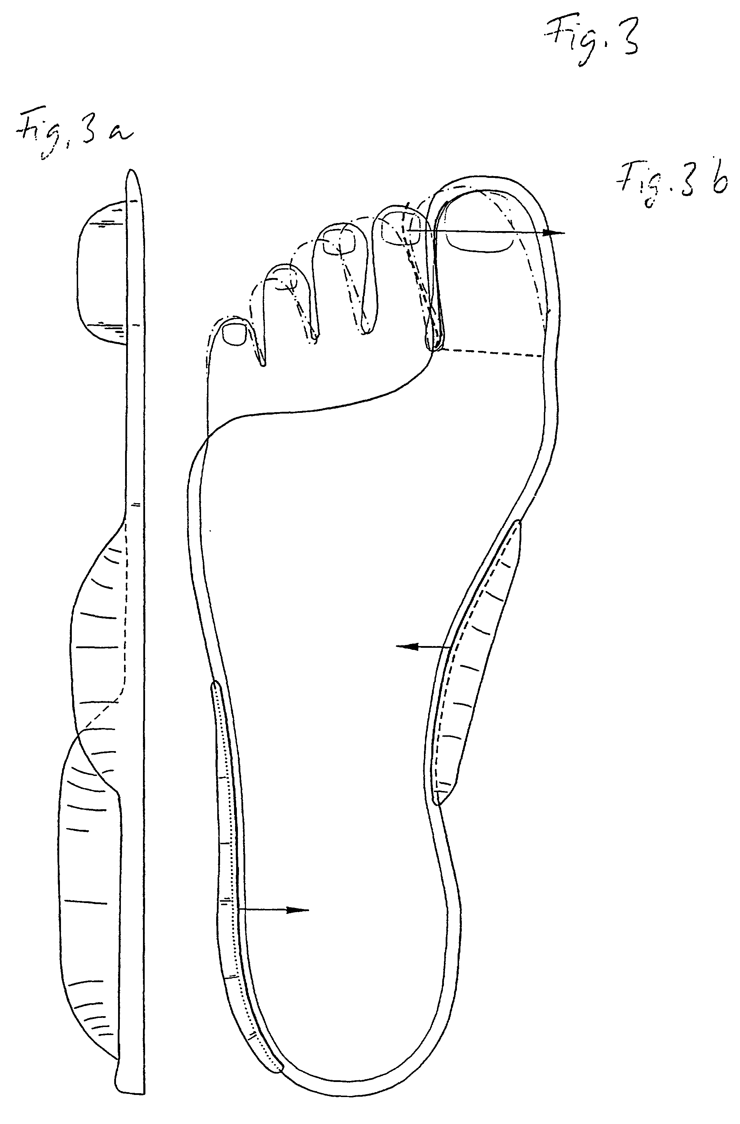

Bei der Ausführungsform gemäß Fig. 3 ist die Basis 1.2 abweichend von den Ausführungsformen gemäß der Figuren 1 und 2 gestaltet. Wie man aus Fig. 3b erkennt, entspricht sie in dieser Draufsicht nicht der Kontur eines Fußes. - Vielmehr ist sie im Bereich der Zehen ausgespart.

Es versteht sich, daß die Höhe eines jeden Korrekturflansches ein solches Maß haben muß, daß eine Korrektur tatsächlich eintritt. In der Praxis kann dies bedeuten, daß der Korrekturflansch wenigstens ein Drittel, besser die Hälfte, und am besten so hoch sein sollte, wie der Durchmesser der großen Zehe ist.

## Patentansprüche

1. Vorrichtung zum Korrigieren einer Fehlstellung einer Zehe bestehend aus einer Sohle (1.2) mit Halteflanschen (1.3, 1.4) und einem dünnen Korrekturflansch (1.1), wobei die Sohle (1.2) bei Benutzung der Vorrichtung parallel zur Fußsohle des Trägers verläuft und die Sohle (1.2), der Korrekturflansch (1.1) und die Halteflansche (1.3, 1.4) aus einem einzigen Kunststoffteil gefertigt sind,
**dadurch gekennzeichnet,**
**daß** der Korrekturflansch (1.1) durch die Zehenlücke zwischen der fehlstehenden und einer Nachbarzehe hindurchgreift, und zwar auf jener Seite der fehlstehenden Zehe, nach der diese geneigt ist, wobei der Korrekturflansch (1.1) derart angeordnet und/oder geneigt ist, daß er der Fehlstellung entgegenwirkt und auf die fehlstehende Zehe im Sinne einer Verschwenkung in die Soll-Stellung einwirkt, und
**daß** die Sohle (1.2) derart gestaltet ist, daß sie zumindest im Bereich der Wurzel der großen Zehe eine Biegung um eine Achse zuläßt, die wenigstens annähernd senkrecht zur Fuß-Längsachse sowie im wesentlichen in der Ebene der Fußsohle verläuft.

2. Vorrichtung nach Anspruch 1, wobei Mittel vorgesehen sind, um zu verhindern, daß der übrige Fuß bei Gebrauch der Vorrichtung die Schwenkbewegung der fehlstehenden Zehe im wesentlichen Maße mitmacht.

3. Vorrichtung nach Anspruch 1 oder 2, wobei die Sohle (1.2) Bestandteil einer Fußbekleidung ist.

## Claims

1. An apparatus for correcting the defective position of a toe, consisting of a sole (1.2) with holding flanges (1.3, 1.4) and a thin corrective flange (1.1), with the sole (1.2) extending parallel to the sole of the wearer's foot when the apparatus is used and the sole (1.2), the corrective flange (1.1) and the holding flange (1.3, 1.4) being made of a single plastic part, **characterized in that** the corrective flange (1.1) reaches through the gap between the defectively positioned toe and an adjacent toe, namely on the side of the defectively positioned toe to which the same is inclined, with the corrective flange (1.1) being arranged and/or inclined in such a way that it counteracts the defective position and acts upon the defectively positioned toe such that it produces a pivoting to the desired position, and that the sole (1.2) is configured in such a way that it allows a bending about an axis at least in the region of the root of the big toe, which bending extends at least approximately perpendicular to the longitudinal axis of the foot and substantially in the plane of the sole of the foot.

2. An apparatus as claimed in claim 1, wherein means are provided in order to prevent that the remainder of the foot joins the pivoting movement of the defectively positioned toe to a relevant extent when the apparatus is used.

3. An apparatus as claimed in claim 1 or 2, wherein the sole (1.2) is the component of footwear.

## Revendications

1. Dispositif pour corriger une déviation d'un orteil, composé d'une semelle (1.2) avec des brides de maintien (1.3, 1.4) et une bride de correction mince (1.1), dans lequel la semelle (1.2) est orientée, pendant l'utilisation du dispositif, parallèlement à la plante du pied du porteur, et la semelle (1.2), la bride de correction (1.1 ) et les brides de maintien (1.3, 1.4) sont fabriquées dans une seule pièce de plastique, **caractérisé en ce que** la bride de correction (1.1) se met en prise à travers l'espace interdigital entre l'orteil dévié et un orteil voisin, sur le côté de l'orteil dévié vers lequel celui-ci est incliné, la bride de correction (1.1) étant disposée et/ou inclinée de sorte qu'elle s'oppose à la déviation et agisse sur l'orteil dévié en le faisant pivoter vers la position nominale, et **en ce que** la semelle (1.2) est conformée de telle sorte qu'elle autorise au moins au niveau de la base du gros orteil une flexion autour d'un axe qui est au moins approximativement perpendiculaire à l'axe longitudinal du pied et passe sensiblement dans le plan de la plante du pied.

2. Dispositif selon la revendication 1, **caractérisé en ce qu'**il est prévu des moyens pour empêcher que le reste du pied suive de manière notable le mouvement de pivotement de l'orteil dévié lors de l'utilisation du dispositif.

3. Dispositif selon la revendication 1 ou 2, **caractérisé en ce que** la semelle (1.2) fait partie d'une chaussure.
